(19) Europäisches Patentamt
European Patent Office
Office européen des brevets

(11) EP 2 255 674 A1

(12) EUROPEAN PATENT APPLICATION
published in accordance with Art. 153(4) EPC

(43) Date of publication:
01.12.2010 Bulletin 2010/48

(51) Int Cl.:
A23J 3/16 (2006.01)    A23L 1/305 (2006.01)
A61K 36/48 (2006.01)   A61K 38/00 (2006.01)
A61P 3/10 (2006.01)    A61P 13/12 (2006.01)

(21) Application number: 09717885.9

(22) Date of filing: 04.03.2009

(86) International application number:
PCT/JP2009/054064

(87) International publication number:
WO 2009/110504 (11.09.2009 Gazette 2009/37)

(84) Designated Contracting States:
AT BE BG CH CY CZ DE DK EE ES FI FR GB GR
HR HU IE IS IT LI LT LU LV MC MK MT NL NO PL
PT RO SE SI SK TR
Designated Extension States:
AL BA RS

(30) Priority: 04.03.2008 JP 2008053033

(71) Applicant: Fuji Oil Company, Limited
Chuo-ku
Osaka-shi
Osaka 542-0086 (JP)

(72) Inventors:
• ASANOMA, Masashi
Izumisano-shi
Osaka 598-8540 (JP)
• KOHNO, Mitsutaka
Izumisano-shi
Osaka 598-8540 (JP)

(74) Representative: Hayes, Adrian Chetwynd
Boult Wade Tennant
Verulam Gardens
70 Gray's Inn Road
London WC1X 8BT (GB)

(54) **SOYBEAN PROTEIN MATERIAL FOR PATIENTS WITH RENAL DISEASE AND FOODS MADE FROM THE SAME**

(57) Provided is a soybean protein material suitable for patients with renal disease which is highly effective in delaying the progression of renal disease, and foods for patients with renal disease made from the soybean protein material. The invention was completed upon obtaining knowledge that, among the acid-precipitable soybean proteins, fractionated soybean proteins obtained by increasing the purity of soy bean acid precipitable proteins other than 7S-globulin and 11S-globulin, specifically, "non-7S and 11S-acid-precipitable proteins," have a strong effect in lowering albumin levels in urine.

Fig. 2

EP 2 255 674 A1

**Description**

Technical Field

The present invention relates to a soybean protein material for patients with renal disease and foods using the same.

Background Art

Recently, due to a sharp increase in the number of patients with diabetes, patients with renal disease which is one of complications are increasing. As a therapeutic approach to the patients who have developed renal disease, a food therapy has a great significance along with a drug therapy. As a typical food therapy, a low protein food therapy of suppressing protein ingestion has been known. In the food therapy using low protein foods, ingestion of proteins per day is generally prescribed as 0.8 to 1.0 g/kg body weight.

In order to protect renal function, the protein ingestion restriction is conducted as described above as the food therapy for the patients with renal disease, but it has been reported that the protein ingestion restriction leads to breakdown of proteins constituting a body, an increase in a nitride compound in blood, and an increase in load on kidney. Also, it is naturally necessary to ingest larger amounts of carbohydrates and fats in order to compensate a shortage of calorie intake. As a result, there are problems of causing obesity and causing various secondary disorders such as a metabolic syndrome.

A protein that has a less load on kidney is considered to be remarkably beneficial for the patients with renal disease since such a protein solves the problems of supplementation of component proteins and calorie intake. It has been reported that a soybean protein (soy protein isolate: SPI) is capable of delaying progression of diabetic renal disease from its initial disease state in experiments of diabetes model animals and patients with type II diabetes as compared to animal proteins (Non-Patent Documents 1, 2, and 3). Also, it has been reported that a well-balanced ingestion of animal proteins and plant proteins can suppress progression of renal disease (Patent Document 1). However, delaying effects of the above document are not sufficient as compared to the low protein therapy, and there is a demand for a protein that exhibits a stronger effect on kidney. Therefore, it is more useful when the stronger effect is exhibited by an ordinary dose of a substance obtained by concentrating a fraction containing active site of a soybean protein.

(References)

Non-Patent Document 1: Sandra R. Teixeira et al., J. Nutr., 133, 673-678, 2003.
Non-Patent Document 2: Joyce Trujillo et al., Am J Physiol Renal Physiol, 288, 1152, F108-F116, 2005.
Non-Patent Document 3: JW Anderson et al., Am. J. Clinical Nutrition, 68, 1347, 1998.
Patent Document 1: JP 2007-176901 A

Disclosure of the Invention

Problems to be Solved by the Invention

Protein ingestion per day is generally prescribed to be 0.8 to 1.0 g/kg body weight in a food therapy. However, the low protein therapy has problems of body protein breakdown, imbalanced high fat diet or high carbohydrate diet, and the like. Though it has been reported that the soybean protein is capable of delaying progression of renal disease as compared to animal proteins, there is a great demand for a new protein material that has a stronger effect. Therefore, an object of the present invention is to find a soybean protein material, which has a high effect of delaying renal disease, for patients with renal disease and to provide foods using the same for patients with renal disease.

Means for Solving the Problems

The inventors have got the idea that a soybean protein is a complex of several proteins, and that an active site may exist in any one of fractions and have searched for the effective fraction, in other words, the inventors have conducted various researches to find a prominent urinary albumin suppression activity in 7S-globulin and 11S-globulin that are currently well known as components of soybean protein as well as in other protein components.

As a result, the inventors have found that a fractionated soybean protein obtained by increasing the purity of a soybean acid-precipitable soybean protein other than 7S-globulin and 11S-globulin, i.e. "non-7S and non-11S acid-precipitable soybean protein" has a strong urinary albumin lowering action to accomplish the present invention.

That is, the present invention is:

(1) A soybean protein material for protein supplementation of patients with renal disease comprising a non-7S and non-11S acid-precipitable soybean protein;

(2) A food for protein supplementation of patients with renal disease comprising a non-7S and non-11S acid-precipitable soybean protein;

(3) A food for delaying renal disease progression in patients with renal disease comprising a non-7S and non-11S acid-precipitable soybean protein as an active ingredient;

(4) A use of a non-7S and non-11S acid-precipitable soybean protein for delaying renal disease progression in patients with renal disease; and

(5) A method for delaying renal disease progression in patients with renal disease comprising using an effective amount of a non-7S and non-11S acid-precipitable soybean protein.

Effect of the Invention

According to the present invention, it is possible to provide a soybean protein material that is highly effective for delaying progression of renal disease as compared to conventional soybean protein isolates and the like and can be ingested by patients with renal disease as a protein source. A fractionated non-7S and non-11S acid-precipitable soybean protein in the present invention does not entail breakdown of proteins constituting a body and imbalanced high fat diet or high carbohydrate diet since ingravescence of the renal disease caused by the fractionated non-7S and non-11S acid-precipitable soybean protein is mild even when ingested in an amount same as that of conventionally studied soybean proteins and enables to provide a useful protein material for patients with renal disease. It is useful for an improvement of QOL (quality of life) of the patients with renal disease that they ingest a food using the fractionated non-7S and non-11S acid-precipitable soybean protein.

Best Mode for Carrying Out the Invention

The present invention is based on the findings that the active site for delaying initial progression of renal disease exists also in a non-7S and non-11S acid-precipitable soybean protein fractionated from a soybean protein, and a soybean protein material for patients with renal disease is obtained by using the non-7S and non-11S acid-precipitable soybean protein. Hereinafter, the present invention will be described in detail.

"Non-7S and non-11S acid-precipitable soybean protein" of the present invention means a soybean protein material obtained by increasing the purity of a minor acid-precipitable soybean protein (hereinafter sometimes abbreviated to "LP" (lipophilic proteins) due to a large amount of associated lipids) other than 7S-globulin and 11S-globulin among acid-precipitable soybean proteins of soybean (hereinafter "non-7S and non-11S acid-precipitable soybean protein" is sometimes referred to as "high LP-SPI"). As used herein, "acid-precipitable soybean protein" means a protein that has a property of being precipitated when the pH of a solution such as a defatted soymilk is adjusted to acidity (pH 4 to 6) among proteins of soybean. Therefore, a protein contained in a soybean protein isolate corresponds to "acid-precipitable soybean protein", and a whey protein which is not precipitated during production of a soybean protein isolate is not included in "acid-precipitable soybean protein".

The "7S-globulin", which is also called "β-conglycinin", means a glycoprotein which is constituted of three types of subunits ($\alpha$', $\alpha$, and $\beta$). The subunits are combined at random to form a trimetric structure. The 7S-globulin has an isoelectric point of near pH 4.8 and a molecular amount of about 170000. Hereinafter, "7S-globulin" is abbreviated to "7S".

Also, "11S-globulin" is also called "glycinin", in which an acidic subunit and a basic subunit are bound by a disulfide binding, and six molecules of these subunits form a hexametric structure. The 11S-globulin has a molecular amount of about 360000. Hereinafter, "11S-globulin" is abbreviated to "11S".

Both of 7S and 11S are typical acid-precipitable soybean proteins contained in soybean and are major storage proteins stored in a protein body. Though there may be fluctuations depending on species, 7S and 11S are components that take up 70% or more of an entire soybean protein in a conventional soybean protein isolate (SPI) in the case where a peak area is measured by staining with Coomassie brilliant blue (CBB) and densitometry in SDS electrophoresis.

In contrast, LP is an aggregate of various acid-precipitable soybean proteins other than 7S and 11S, and, since substantial properties of LP have not been well recognized due to a property of LP of hardly being stained with CBB in the SDS electrophoresis as compared to 7S and 11S, it has been difficult to attain accurate quantification with the method.

Therefore, it is possible to employ, as a simplified method, the following method of selecting major proteins from each of proteins of 7S, 11S, and LP and estimating an LP content from staining ratios of the selected proteins.

[Method for Estimating LP Content]

(a) As the major proteins of each of the proteins: the $\alpha$ subunit and $\alpha'$ subunit ($\alpha+\alpha'$) are selected from 7S; the acidic subunit (AS) is selected from 11S; and 34kDa protein and lipoxygenase (P34+Lx) are selected from LP, and staining ratios of the selected proteins are detected by SDS-PAGE. It is possible to conduct the electrophoresis under the conditions shown in Table 1.
(b) X (%) is calculated:

$$X (\%) = (P34+Lx)/\{(P34+Lx)+(\alpha+\alpha')+AS\} \times 100\%.$$

(c) Since an LP content of an isolated protein prepared from a low modified defatted soybean measured by the above methods 1 and 2 before heat sterilization becomes about 38%, (P34+Lx) is multiplied by a correction coefficient k*=6 to obtain X=38(%).
(d) That is, a lipophilic proteins content index (hereinafter abbreviated to LCI) is calculated from the following expression.

(Table 1)

| | |
|---|---|
| Applied amount: | 10 $\mu$l of 0.1% protein sample solution in each of wells |
| Well width: | 5 mm |
| Well capacity: | 30 $\mu$l |
| Staining liquid: | 1 g of Coomassie brilliant blue (CBB), 500 ml of methanol, 70 ml of glacial acetic acid (after perfectly dissolving CBB into methanol, acetic acid and water are added to obtain 1L of the staining liquid) |
| Staining period: | 15 hours |
| Decoloration period: | 6 hours |
| Densitometer: | GS-710 Calibrated Imaging Densitometer/Quantity One Software Ver. 4.2.3 (Bio Rad Japan Co., Ltd.), Scanning width: 5.3 mm, Sensitivity: 30 |

(Expression 1)

$$LCI(\%) = k^* \times (P34+Lx)/ \{k^* \times (P34+Lx)+(\alpha+\alpha')+AS\} \times 100$$

k*: correction coefficient (6)
P34: LP major component, 34kDa protein
Lx: LP major component, lipoxygenase
$\alpha$: 7S major component, $\alpha$ subunit
$\alpha'$: 7S major component, $\alpha'$ subunit
AS: 11S major component, acidic subunit

An LCI value of high LP-SPI is preferably 50% or more and more preferably 60% or more in order to attain high LP purity. Since the LCI value of ordinary soybean protein isolates is about 40%, proteins having such a low level LCI value can not be included in the high LP-SPI.

Hereinafter, preparation examples of the high LP-SPI which is the novel soybean protein of the present invention will be described. The high LP-SPI preparation method is not particularly limited insofar as the method enables to concentrate LP. For example, in the case of fractionating the high LP-SPI directly from whole fat soybeans, it is possible to obtain the high LP-SPI by extracting a soymilk at 4°C to 60°C, collecting an emulsified layer by centrifuging the soymilk, and recovering a protein contained in the emulsified layer as an LP fraction.

Also, it is possible to obtain the high LP-SPI by eliminating a fraction that is rich in 7S or a fraction that is rich in 11S and 7S from a soybean protein solution containing 7S and LP or 7S, 11S, and LP (soymilk, soybean protein isolate solution, 11S-eliminated fractionated soybean protein solution, etc.). In the case of extracting a soymilk from defatted soybeans or the like, a temperature is preferably 4°C to 60°C, and a pH is preferably 6 to 9.

As a more specific example of the above-described method, it is possible to conduct a method of preparing beforehand a protein-containing liquid rich in 7S and LP by eliminating a fraction rich in 11S and eliminating the fraction rich in 7S. As a method for eliminating the fraction rich in 11S beforehand, it is possible to employ a known method of adjusting pH to 5.8 to 6.4 which is the isoelectric point of 11S and selectively precipitating and eliminating 11S, and it is possible to employ or appropriately combine the following fractionating methods for the preparation.

(1) 11S Elimination method according to Thahn, V. H. and Shibasaki, K., J. Agric. Food Chem., 24, 117(1976));
(2) Method of extracting a soymilk near the isoelectric point of 11S (JP 55-124457 A);
(3) Method of eliminating a fraction rich in 11S by adding a small amount of a calcium salt during extraction (JP 48-56843 A);
(4) Method of producing by a method of insolubilizing a fraction rich in 11S in the presence of sodium chloride or potassium chloride of pH 1.2 to 4.0 and eliminating the fraction (JP 49-31843 A);
(5) Method of separating and eliminating a 11S fraction by adjusting a slurry obtained by isoelectric precipitation to pH 5.0 to 5.6 and adjusting a sodium chloride concentration to a molar concentration of 0.01 to 0.2 M (JP 58-36345 A);
(6) Method utilizing a phenomenon that solubility of 11S-globulin is decreased at a low temperature (cryo precipitation phenomenon), which consists of eliminating an insolubilized fraction that is rich in 11S by treating a soybean protein material in the presence of a glutathione compound or a cysteine compound and in a water system of pH 6.5 or more and adjusting a pH range and a temperature range within pH 5.5 to 7.0 and 20°C or less (JP 61-187755 A);
(7) Method of eliminating an insolubilized fraction rich in 11S by reacting a solution containing a soybean protein with phytase and adjusting pH of the solution to 5.6 to 6.6 (WO00/58492);
(8) Method of performing water extraction of a soybean protein from soybeans that lack 11S; and the like.

The fractionated soybean protein from which 11S is eliminated has been subjected to many experiments as a soybean protein rich in 7S. However, in any one of the experiments, the existence of LP is not suggested because of the difficulty in staining LP in the SDS electrophoresis. The inventors have used the 7S fractionated soybean protein and the 11S fractionated soybean protein that have high purity, i.e., these are less contaminated with LP, and the high LP-SPI having the high LP purity as samples and have clarified the original functions of the proteins.

The fraction obtained by the above-described method is a fraction rich in 7S and LP, in which LP is concentrated to a certain degree, but preferred as the high LP-SPI to be used in the present invention is a fraction obtained by further eliminating a fraction rich in 7S from the fraction and increasing purity of LP to a higher level. As a method of attaining the high purity of LP, a method of raising the temperature to 40°C to 65°C within a pH range of 4 to 5.5 and recovering an insolubilized fraction generated within a pH range of 5.3 to 5.7 (see WO2006/129647), a method of selectively solubilizing 7S by increasing the concentration of a salt such as sodium salt, and the like can be employed. It is possible to obtain the high LP-SPI by recovering the thus-generated insolubilized fraction rich in LP and eliminating the insolubilized fraction rich in 7S.

The inventors have prepared the high LP-SPI having high LP purity from a soybean protein material and have fed a mouse of renal disease onset model with the high LP-SPI to confirm an effect of the high LP-SPI on a mouse urinary albumin concentration. As a result, it have been confirmed that a stronger urinary albumin increase suppression effect is exhibited by ingesting the high LP-SPI as a protein source as compared to conventional soybean protein isolates, 7S fractionated soybean proteins, and 11S fractionated soybean proteins, thereby acquiring findings that the high LP-SPI is effective for delaying progression of renal disease of patients with renal disease and is a remarkably effective protein material as a protein supplementation source.

It is possible to obtain protein supplementation foods for patients with renal disease by preparing foods of various forms by using an effective amount of the high LP-SPI. It is possible to use the foods for delaying progression of renal disease of patients with renal disease. A use amount of the high LP-SPI in the foods can appropriately be set depending on a degree of disease, sex, age, and the like of a patient with renal disease and can be set as the effective amount in such a manner that a predetermined ratio (e.g. from 5% to 100%) of the required protein ingestion per day is derived from the high LP-SPI.

It is possible to use in combination a material that has an effect of delaying or curing renal diseases in the foods of the present invention in addition to the use of the high LP-SPI.

As types of the foods of the present invention, it is possible to blend the high LP-SPI with foods in general forms including soft drink, powdered drink, dairy product, soymilk, fermented soymilk, soybean protein drink, protein powder, tofu (bean curd), natto, abura-age (thin fried bean curd), atsu-age (thick fried bean curd), gammodoki (fried bean curd cake containing vegetables and other ingredients), hamburger, meatball, fried chicken, nugget, various prepared foods, confectionery such as baked confectionery, protein bar, serial, candy, chewing gum, and jelly, tablet, bread, steamed rice, and the like. Further, it is possible to provide the foods using the high LP-SPI as an active ingredient (contributing ingredient) as foods for healthcare (food for specified health use, and the like) by stating on a package, pamphlet, or the like of the foods that the foods have various efficacies and effects related to a reduction in urinary albumin.

Examples

Hereinafter, examples of the present invention will be described.

[Experimental Example] Confirmation of Initial Progression Delaying Action on Diabetic Renal Disease

An initial progression delaying effect on diabetic renal disease of each of soybean proteins was confirmed by using materials of a high LP-SPI (LCI value: 71%), a 11S fractionated soybean protein (LCI value: 12%), and a 7S fractionated soybean protein (LCI value: 12%) prepared in accordance with methods of Example 2 of Comparative Experimental Example 2, Comparative Example 3, Example 7, and Example 9 in WO2006/129647 and a commercially available soybean protein isolate (LCI value: 40%) as samples.

Based on AIN-93G composition (REEVES P. G. et al., J. NUTR., 123, 1939-1951, 1993), a mixed food containing as a crude protein amount 20 wt% of casein "Vitamin-Free Casein" (manufactured by Oriental Yeast Co., Ltd., hereinafter described as "casein") was used as a control, and the control and experimental foods (Table 2) prepared by substituting each of protein sources thereof by an soybean protein isolate (Comparative Example 1), a 11S fractionated soybean protein (Comparative Example 2), a 7S fractionated soybean protein (Comparative Example 3), or a high LP-SPI (Example 1) were fed to animals by the following method. The model animals were 50 KKAy-strain male mice of 8-week old (marketed by CLEA Japan, Inc.). After one week of preliminary breeding, the mice were sorted into groups in such a manner that each of the groups consists of 10 mice and that average weights and urinary albumin amounts of the groups were substantially the same, and experimental feeding was conducted for 7 weeks.

(Table 2)

| | Casein group | SPI group | 7S group | 11s group | High LP-SPI group |
|---|---|---|---|---|---|
| | | | | | (Unit: g) |
| Casein | 22.7 | | | | |
| Soybean protein isolate | | 23.4 | | | |
| 7S | | | 24.9 | | |
| 11S | | | | 22.0 | |
| LP | | | | | 24.8 |
| Soybean oil | 6.0 | 6.0 | 6.0 | 6.0 | 6.0 |
| Lard | 4.0 | 4.0 | 4.0 | 4.0 | 4.0 |
| Sucrose | 10.0 | 10.0 | 10.0 | 10.0 | 10.0 |
| β corn starch | 34.3 | 33.7 | 32.2 | 35.0 | 32.2 |
| α corn starch | 13.2 | 13.2 | 13.2 | 13.2 | 13.2 |
| Cellulose | 5.0 | 5.0 | 5.0 | 5.0 | 5.0 |
| Mineral mixture (AIN-93G composition) | 3.5 | 3.5 | 3.5 | 3.5 | 3.5 |
| Vitamin mixture (AIN-93 composition) | 1.0 | 1.0 | 1.0 | 1.0 | 1.0 |
| Choline bitartrate | 0.25 | 0.25 | 0.25 | 0.25 | 0.25 |
| Total | 100.0 | 100.0 | 100.0 | 100.0 | 100.0 |

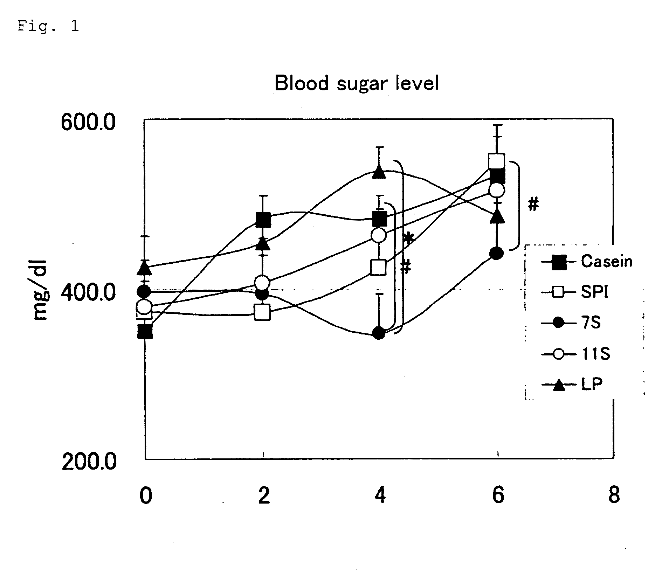

Blood was collected from a tail of each of the mice every 2 weeks at 9 am under non-fastening conditions during the experiment period. The blood was treated with heparin and subjected to 15 minutes of centrifugation at 3000 rpm, and the obtained blood plasma was used as a blood sample for a measurement of a blood sugar level by using Fuji Dri-Chem 7000 (manufactured by Fujifilm Corporation). The blood sugar levels detected every 2 weeks were shown in Table 3 and Fig. 1. "LP" in Fig. 1 was the high LP-SPI group.

(Table 3) Changes in Blood Sugar Levels of Mice Detected Every 2 Weeks

| | | Casein | SPI | 7S | 11S | High LP-SPI |
|---|---|---|---|---|---|---|
| | n | 8 | 9 | 10 | 10 | 8 |
| 0W | mg/dl | 352 ± 28 | 373 ± 47 | 397 ± 36 | 379 ± 30 | 425 ± 37 |
| 2W | mg/dl | 482 ± 28 | 372 ± 37 | 395 ± 65 | 406 ± 33 | 454 ± 29 |

(continued)

|  |  | Casein | SPI | 7S | 11S | High LP-SPI |
|---|---|---|---|---|---|---|
|  | n | 8 | 9 | 10 | 10 | 8 |
| 4W | mg/dl | 483 ± 27 | 424 ± 32 | 347 ± 46# | 462 ± 31 | 538 ± 29 |
| 6W | mg/dl | 533 ± 47 | 549 ± 44 | 440 ± 61# | 516 ± 28 | 485 ± 65 |
| Values are means ± SE #: 0.05 < p < 0.1 by T-test (vs Casein). | | | | | | |

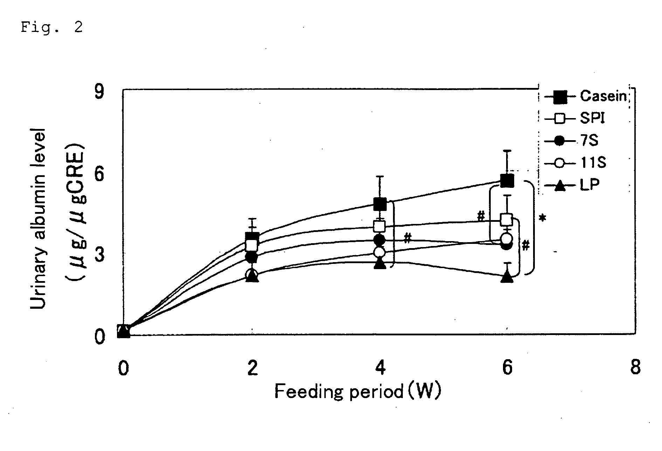

Also, the mice were placed in a metabolism cage every 2 weeks of the feeding period for 24-hour urine collection. By using the obtained urine, a creatinine (CRE) concentration and an albumin (ALB) concentration were measured, and a ratio (ALB/CRE) was calculated. The albumin/creatinine values detected every 2 weeks were shown in Table 4 and Fig. 2. "LP" in Fig. 2 was the high LP-SPI group.

(Table 4) Changes in Urinary Albumin Levels of Mice Detected Every 2 Weeks

|  |  | Casein | SPI | 7S | 11S | High LP-SPI |
|---|---|---|---|---|---|---|
|  | n | 8 | 9 | 10 | 10 | 8 |
| 0W | μg/mg CRE | 0.17 ± 0.05 | 0.16 ± 0.06 | 0.17 ± 0.05 | 0.17 ± 0.05 | 0.18 ± 0.05 |
| 2W | μg/mg CRE | 3.52 ± 0.73 | 3.25 ± 0.68 | 2.83 ± 0.70 | 2.39 ± 0.52 | 2.15 ± 0.47 |
| 4W | μg/mg CRE | 4.80 ± 1.00 | 3.92 ± 0.71 | 3.43 ± 0.81 | 2.98 ± 0.48 | 2.62 ± 0.58* |
| 6W | μg/mg CRE | 5.62 ± 1.10 | 4.18 ± 0.94 | 3.26 ± 0.58 | 3.49 ± 0.80 | 2.12 ± 0.45* |
| Values are means ± SE. *: p< 0.05 by T-test (vs Casein). #: 0.05 < p < 0.1 by T-test (vs Casein). | | | | | | |

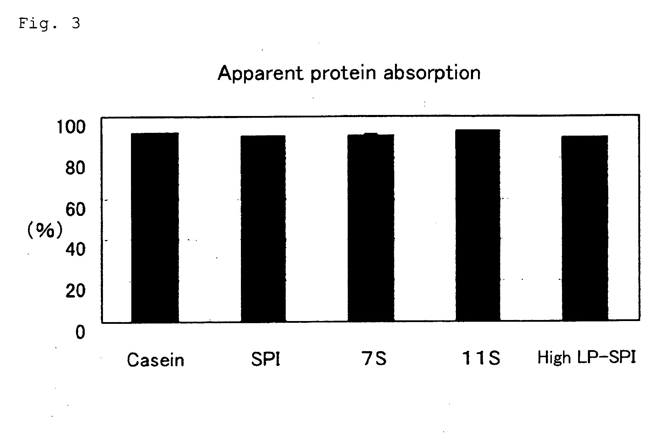

Feces were collected for 4 days at 6th week of the feeding period to measure a nitride content in the feces. An apparent protein absorption was calculated by comparing a nitride content in the ingested feed and the nitride content in the feces. The apparent protein absorptions were shown in Table 5 and Fig. 3.

(Table 5) Apparent Protein Absorption Calculated from Ingested Nitride Content and Egested Nitride Content

|  |  | Casein | SPI | 7S | 11S | High LP-SPI |
|---|---|---|---|---|---|---|
|  | n | 10 | 10 | 10 | 10 | 10 |
| Ingested amount | g | 5.5 ± 0.42 | 6.0 ± 0.13 | 5.8 ± 0.26 | 6.1 ± 0.26 | 5.2 ± 0.45 |
| Ingested N amount | g | 0.17 ± 0.014 | 0.19 ± 0.004 | 0.19 ± 0.008 | 0.19 ± 0.008 | 0.17 ± 0.015 |
| Feces amount | g | 0.56 ± 0.05 | 0.72 ± 0.02 | 0.61 ± 0.03 | 0.65 ± 0.03 | 0.58 ± 0.06 |
| Egested N amount | g | 0.013 ± 0.002 | 0.019 ± 0.001 | 0.018 ± 0.001 | 0.015 ± 0.000 | 0.021 ± 0.001 |
| Apparent protein absorption | % | 92.1 + 0.2 | 90.3 ± 0.2 | 90.6 ± 0.2 | 92.6 ± 0.2 | 89.3 ± 0.2 |
| Values are means ± SE | | | | | | |

As is apparent from the above results, the ingestion of the high LP-SPI produces the more prominent urinary albumin increase suppression action as compared to the conventional soybean protein isolate and the 7S and 11S fractionated soybean proteins. That is, it is confirmed that the high LP-SPI fractionated by the inventors is the novel material having a high progression delaying action for the initial stage of renal disease.

As to the blood sugar level, since the 7S fraction attained the lowest level, and since the blood sugar level lowering effect of the high LP-SPI fraction is not confirmed, it is revealed that the renal disease progression delaying effect of the high LP-SPI is not attributable to improvement in blood sugar level.

Also, though the protein absorption of the high LP-SPI fraction is very high like the conventional soybean protein, the high LP-SPI exhibited the renal disease delaying effect. Therefore, it is confirmed that the novel soybean protein material is the protein that is remarkably useful for the patients with renal disease.

Brief Description of the Drawings

[Fig. 1] Fig. 1 is a graph showing the results of Table 1 indicating the changes over time of the blood sugar levels of the mice detected every 2 weeks.

[Fig. 2] Fig. 2 is a graph showing the results of Table 2 indicating the changes over time of the urinary albumin values of the mice detected every 2 weeks.

[Fig. 3] Fig. 3 is a graph showing the results of Table 3 indicating the apparent protein absorptions calculated from the ingested nitride contents and egested nitride contents.

**Claims**

1.  A soybean protein material for protein supplementation of patients with renal disease comprising a non-7S and non-11S acid-precipitable soybean protein.

2.  A food for protein supplementation of patients with renal disease comprising a non-7S and non-11S acid-precipitable soybean protein.

3.  A food for delaying renal disease progression in patients with renal disease comprising a non-7S and non-11S acid-precipitable soybean protein as an active ingredient.

4.  A use of a non-7S and non-11S acid-precipitable soybean protein for delaying renal disease progression in patients with renal disease.

5.  A method for delaying renal disease progression in patients with renal disease comprising using an effective amount of a non-7S and non-11S acid-precipitable soybean protein.

Fig. 1

Blood sugar level

Fig. 2

Fig. 3

## Apparent protein absorption

## INTERNATIONAL SEARCH REPORT

| International application No. |
| --- |
| PCT/JP2009/054064 |

A.  CLASSIFICATION OF SUBJECT MATTER
*A23J3/16*(2006.01)i, *A23L1/305*(2006.01)i, *A61K36/48*(2006.01)i, *A61K38/00*
(2006.01)i, *A61P3/10*(2006.01)i, *A61P13/12*(2006.01)i

According to International Patent Classification (IPC) or to both national classification and IPC

B.  FIELDS SEARCHED

Minimum documentation searched (classification system followed by classification symbols)
A23J3/16, A23L1/305, A61K36/48, A61K38/00, A61P3/10, A61P13/12

Documentation searched other than minimum documentation to the extent that such documents are included in the fields searched
| | | | |
| --- | --- | --- | --- |
| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)
JSTPlus/JMEDPlus/JST7580(JDreamII)

C.  DOCUMENTS CONSIDERED TO BE RELEVANT

| Category* | Citation of document, with indication, where appropriate, of the relevant passages | Relevant to claim No. |
| --- | --- | --- |
| X/Y | WO 2006/129647 A1  (Fuji Oil Co., Ltd.), 07 December, 2006 (07.12.06), Particularly, Par. No. [0027] & EP 1905312 A1          & CN 101184403 A | 1/2,3 |
| Y | TEIXEIRA, S.R. et al, Isolated Soy Protein Consumption Reduces Urinary Albumin Excretion and Improves the Serum Lipid Profile in Men with Type 2 Diabetes Mellitus and Nephropathy, J Nutr, 2004, Vol.134 No.8 Page.1874-1880 | 2,3 |
| Y | Takanobu SAKEMI et al., "Shizen Hassho Koshikessho Rat no Jinshogai Shinten ni Oyobosu Daizu Tanpakushitsu to Daizu Tanpakushitsu Alcohol Chushutsubutsu (SPE) no Eikyo", Soy Protein Research, Japan, 2002, No.5, pages 76 to 80 | 2,3 |

|☒| Further documents are listed in the continuation of Box C. | |☐| See patent family annex. |
| --- | --- | --- | --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 01 June, 2009 (01.06.09) | 09 June, 2009 (09.06.09) |

| Name and mailing address of the ISA/ Japanese Patent Office | Authorized officer |
| --- | --- |
| Facsimile No. | Telephone No. |

Form PCT/ISA/210 (second sheet) (April 2007)

**INTERNATIONAL SEARCH REPORT**

| International application No. |
| --- |
| PCT/JP2009/054064 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | JP 2007-176901 A  (Kabushiki Kaisha USCure),<br>12 July, 2007 (12.07.07),<br>(Family: none) | 1-3 |
| P,A | WO 2008/069273 A1  (Fuji Oil Co., Ltd.),<br>12 June, 2008 (12.06.08),<br>(Family: none) | 1-3 |
| P,A | WO 2009/060678 A1  (Fuji Oil Co., Ltd.),<br>14 May, 2009 (14.05.09),<br>(Family: none) | 1-3 |

Form PCT/ISA/210 (continuation of second sheet) (April 2007)

## INTERNATIONAL SEARCH REPORT

| | International application No. |
|---|---|
| | PCT/JP2009/054064 |

---

**Box No. II    Observations where certain claims were found unsearchable (Continuation of item 2 of first sheet)**

This international search report has not been established in respect of certain claims under Article 17(2)(a) for the following reasons:

1. ☒ Claims Nos.: 4,5
because they relate to subject matter not required to be searched by this Authority, namely:
A method for delaying the progression of nephropathy in a nephropathy patient pertains to a method for the treatment of a human body, and therefore relates to a subject matter which this international searching authority is not required to search.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

---

**Box No. III    Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**     ☐ The additional search fees were accompanied by the applicant's protest and, where applicable,
the                          payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007176901 A
- JP 55124457 A
- JP 48056843 A
- JP 49031843 A
- JP 58036345 A
- JP 61187755 A
- WO 0058492 A
- WO 2006129647 A

**Non-patent literature cited in the description**

- **Sandra R. Teixeira et al.** *J. Nutr.,* 2003, vol. 133, 673-678
- **Joyce Trujillo et al.** *Am J Physiol Renal Physiol,* 2005, vol. 288 (1152), F108-F116
- **JW Anderson et al.** *Am. J. Clinical Nutrition,* 1998, vol. 68, 1347
- **Thahn, V. H. ; Shibasaki, K.** *J. Agric. Food Chem.,* 1976, vol. 24, 117
- **REEVES P. G. et al.** *J. NUTR.,* 1993, vol. 123, 1939-1951